# EUROPEAN PATENT APPLICATION

(11) **EP 1 518 537 A2**
(43) Date of publication of application: **30.03.2005**
(21) Application number: 04255924.5
(22) Date of filing: 28.09.2004
(51) Int. Cl.: A61K 7/06

(54) **Hair conditioning compositions**

(30) Priority: 29.09.2003 US 675868
(71) Applicant: NEUTROGENA CORPORATION, Los Angeles California 90045-5544 (US)
(72) Inventor: Lim, Connie, Woodland Hills, CA 91367 (US); Hornby, Sidney, San Pedro, CA 90731 (US); Greenway, Jennifer, Los Angeles, CA 90036 (US)
(74) Representative: James, Anthony Christopher W.P.

(57) **Abstract**

The present invention features a composition for application to the hair including: (i) a first hair conditioning agent, wherein such first hair conditioning agent penetrates into the core of the hair; (ii) a second hair conditioning agent, wherein such second hair conditioning agent penetrates into the cortex region of the hair but does not substantially penetrate into the core of the hair; and (iii) a third hair conditioning agent, wherein such third hair conditioning agent remain on the hair surface and does not substantially penetrate into the cortex of the hair.

## Description

### FIELD OF THE INVENTION

The present invention relates to compositions for conditioning the hair.

### BACKGROUND OF THE INVENTION

Washing and conditioning the hair is an integral part of the personal hygiene routine. Hair care products are often expected to deliver more than just cleansing and detangling, such as conditioning. Conditioning benefits are best delivered via a mix of ingredients selected to provide both core strengthening as well as surface enhancement. Materials that penetrate completely through the hair cross section have been associated with improved softness and strength. However, for the most effective conditioning, it is also necessary to have ingredients that deposit on the surface to impart manageability and shine. Materials that can penetrate just through the cuticle layer and into the outer cortex will help to reinforce the hair cuticle scale structure. If the cuticle has better adhesion to the rest of the hair fiber it is less likely to lift up, curl and chip, thus maintaining the smooth texture of the hair and helping to prevent surface damage that may lead to split ends.

The present invention relates to products that contain a novel blend of these three classes of hair conditioning agents.

### SUMMARY OF THE INVENTION

In one aspect, the present invention features a composition for application to the hair including: (i) a first hair conditioning agent, wherein such first hair conditioning agent penetrates into the core of the hair; (ii) a second hair conditioning agent, wherein such second hair conditioning agent penetrates into the cortex region of the hair but does not substantially penetrate into the core of the hair; and (iii) a third hair conditioning agent, wherein such third hair conditioning agent remain on the hair surface and does not substantially penetrate into the cortex of the hair. In another aspect, the present invention features a method for conditioning the hair by applying to the hair the above composition.

In another aspect, the present invention relates to a product including the above composition wherein the product includes advertising stating that the first hair conditioning agent, the second hair conditioning agent, and said third hair conditioning agent condition different regions of the hair.

In another aspect, the present invention features a method of promoting the above composition wherein the method includes promoting that the first hair conditioning agent, the second hair conditioning agent, and the third hair conditioning agent condition different regions of the hair.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Unless otherwise indicated, a percentage of an ingredient refers to a percentage by weight (e.g., %W/W).

What is meant by a "product" is a product in finished packaged form. In one embodiment, the package is a container such as a bottle, a pump, a jar, or a tube containing the composition. The product may further contain additional packaging such as a plastic or cardboard box for storing such container. In one embodiment, the product contains instructions directing the user to use the composition on the hair (e.g., as a shampoo, hair styling or finishing product, perming or body wave product, hair coloring product, a rinse-off conditioner, or a leave-on conditioner). Such instructions may instruct the user to apply the composition by massaging or rubbing the composition into the hair and, if the product is not be left on the hair, to rinse the hair with water following application. In one embodiment, the product contains advertising stating that the first hair conditioning agent, the second hair conditioning agent, and the third hair conditioning agent condition different regions of the hair (e.g., different layers of the hair such as the center, middle, and outer layers of the hair).

What is meant by "promoting" is promoting, advertising, or marketing. Examples of promoting include, but are not limited to, written, visual, or oral statements made on the product or in stores, magazines, newspaper, radio, television, Internet, and the like. In one embodiment, the product contains advertising stating that the first hair conditioning agent, the second hair conditioning agent, and the third hair conditioning agent condition different regions of the hair. Examples of such statements include, but are not limited to: helps moisturize or condition all three layers of the hair; the first hair conditioning agent helps moisturize or condition the center, inner most layer, inner cortex, medulla, or core of the hair; the second hair conditioning agent helps moisturize or condition the middle layer, middle region, or cortex of the hair; and the third hair conditioning agent helps moisturize, condition, or promotes cuticle adhesion of the outer layer or surface of the hair.

As used herein, "topical application" means directly laying, spreading, or spaying on the hair using, e.g., by use of the hands or an applicator.

As used herein, "cosmetically-acceptable" means that the cosmetically active agents or inert ingredients which the term describes are suitable for use in contact with tissues (e.g., the skin) without undue toxicity, incompatibility, instability, irritation, allergic response, and the like, commensurate with a reasonable benefit/risk ratio.

As used herein, "safe and effective amount" means an amount of compound or composition sufficient to significantly induce a positive modification in the condition to be regulated or treated, but low enough to avoid serious side effects. The safe and effective amount of the compound or composition will vary with the particular condition being treated, the age and physical condition of the end user, the severity of the condition being treated/prevented, the duration of the treatment, the nature of concurrent therapy, the specific compound or composition employed, the particular cosmetically-acceptable topical carrier utilized, and like factors.

### Hair Conditioning Agents

The composition of the present invention includes (i) one or more first hair conditioning agents that penetrate into the core of the hair; (ii) one or more second hair conditioning agents that penetrate into the cortex region of the hair but do not substantially penetrate into the core of the hair; and (iii) one or more third hair conditioning agents that remain on the hair surface and does not substantially penetrate into the hair. What is meant by a "hair conditioning agent" is an ingredient which conditions hair. What is meant by "condition" is to enhance the appearance of hair, enhance the feel of hair, increase hair body, increase hair suppleness, facilitate hair styling, improve gloss of hair, moisturize the hair, improve sheen of hair, and/or improve the texture of hair (e.g., that has been damaged by chemical treatment, environmental effects, or physical action). Examples of hair conditioning agents, include but are not limited to, those recited in the International Cosmetic Ingredient Dictionary and Handbook, pages 2912-2920 (The Cosmetic, Toiletry, and Fragrance Association, Ninth Edition 2002).

What is meant by "penetrate" is that the hair conditioning agent is deposited into a region of the hair following application of the conditioning agent to the hair for twenty-four hours. Such analysis of hair penetration is set forth below in Example 8.

In one embodiment, one or more of the hair conditioning agents are oils or compounds derived from oils (including but not limited to esters of the fatty acids of such oils). Examples of such derivatives include, but are not limited to, PEG-7 olivate, meadowfoam estolide, and almond oil PEG-6 esters

Examples of first hair conditioning agents include, but are not limited to, avocado oil, apricot kernel oil, olive oil, sesame oil, and coconut oil, and derivatives thereof such as PEG-7 olivate. Examples of second hair conditioning agents include, but are not limited to, meadowfoam seed oil and derivatives thereof such as meadowfoam estolide. Examples of third hair conditioning agents include, but are not limited to, PEG-8/SMDI copolymer, palmitoyl myristyl serinate, jojoba oil, mineral oil, sunflower oil, almond oil, and proteins such as silk proteins, wheat proteins, and almond proteins, and derivatives thereof such as almond oil PEG-6 esters.

In one embodiment, the composition includes from about 0.001 to about 99 percent, by weight, of each of the hair conditioning agents. If the product is to be used as a shampoo, it will typically include from about 0.01 to about 10 percent, e.g., about 0.1 to about 1 percent, by weight of each of the hair conditioning agents. If the product is to be used as a leave-on conditioner, it will typically include from about 0.01 to about 10 percent, e.g., about 0.1 to about 1 percent, by weight of each of the hair conditioning agents. If the product is to be used as a rinse-off conditioner, it will typically include from about 0.01 to about 10 percent, e.g., about 0.1 to about 1 percent, by weight of each of the hair conditioning agents. If the product is to be used as a rinse-off oil treatment product, it will typically include from about 0.01 to about 99 percent, e.g., about 0.1 to about 50 percent, by weight of each of the hair conditioning agents.

### Foaming Surfactants

In one embodiment, the composition of the present invention is to be used as a shampoo or a conditioning foam that includes one or more foaming surfactant(s). Examples of foaming surfactants are found on pages 1673-89 of the International Cosmetic Ingredient Dictionary and Handbook (7^{th} Ed., The Cosmetic, Toiletry, and Fragrance Association, Washington, DC, 1997), hereinafter the ICI Handbook. In one embodiment, the foaming surfactant is an anionic, amphoteric, or nonionic surfactant. Examples of anionic foaming surfactants include, but are not limited to, sodium, magnesium, potassium, or ammonium salts of alkyl acyl taurates, alkyl sulfates, alkyl ether sulfates, olefin sulfonates, acyl isethionates, acyl sarcosinates, alkyl sulfosuccinates, and C₈-C₂₂ fatty acids. Specific examples of anionic surfactants include, but are not limited to, sodium cocoyl sarcosinate, sodium cocoyl isethionate, sodium trideceth sulfate, disodium laureth sulfosuccinate, sodium lauroyl lactylate, sodium laurate, sodium myristate, sodium palmitate, sodium methyl cocoyl taurate, and sodium lauroyl sarcosinate.

Examples of amphoteric and nonionic foaming surfactants include, but are not limited to, sodium cocoampho(di)acetate, sodium laurampho(di)acetate, sodium cocoamphopropionate, cocamidopropyl betaine, alkylpolyglucosides, and poloxamers.

In one embodiment, the composition includes about 0.1 to about 70 percent, by weight, of said at least one foaming surfactant, e.g., about 0.5 to about 15 percent, by weight of the at least one foaming surfactant.

### Hair Colorants

In one embodiment, the composition contains a hair colorant or dye for coloring the hair (e.g., temporary, semi-permanent, or permanent hair color). Examples of hair colorants and dyes are well known in the art. See, e.g., U.S. Patent Nos. 6,432,147, 6,616,709, and 6,599,330, U.S. Patent Application 2001/039,685, and European Patent Nos. 1,279,395 and 664,114.

### Other Ingredients

The composition may further comprise one or more chelating agents such as disodium EDTA, sunscreens, pH adjusters such as citric acid and sodium citrate, vitamins such as vitamins A, Bs, C, and E, fragrances, and preservatives such as parabens and phenoxyethanol.

### Examples

The following is a description of the manufacture of compositions of the present invention. Other compositions of the invention can be prepared in an analogous manner by a person of ordinary skill in the art.

### Example 1:

Table I an example of a formulation for a conditioning shampoo.

**Table 1**

| **Component INCI Name** | **Supplier** | **% W/W** | **% W/W Range** |
|---|---|---|---|
| Water | | 54.29 | 0.1 - 70 |
| Guar Hydroxypropyltrimon ium Chloride | Rhodia Inc, Cranbury, NJ | 0.5 | 0.01 - 5 |
| Guar (Cyamopsis Tetragonoloba) Gum | Rhodia Inc, Cranbury, NJ | 0.3 | 0.01 - 5 |
| Citric Acid | Tate and Lyle Decatur, IL | 0.65 | 0.01 - 2 |
| Sodium Trideceth Sulfate (and) Sodium Lauroamphoacetate | Rhodia Inc, Cranbury, NJ | 35 | 0.1 - 70 |
| Methylparaben | Ueno Fine Chemicals, New York, NY | 0.3 | 0.01 - 1 |
| Propylparaben | Ueno Fine Chemicals, New York, NY | 0.2 | 0.01 - 1 |
| Cetyl Alcohol | Cognis Corporation, Ambler, PA | 0.5 | 0.01 - 10 |
| Cocamide MEA | Uniqema, Paterson, NJ | 1 | 0.01 - 10 |
| Sodium Chloride | Cargill, Newark, NJ | 3.5 | 0.1 - 5 |
| Meadowfoam (Limnanthes Alba) Seed Oil | Botagenics, Inc. Northridge, CA | 0.2 | 0.01 - 10 |
| Olive (Olea Europaea) Fruit Oil | Croda, Mill Hall, PA | 0.2 | 0.01 - 10 |
| Panthenyl Ethyl Ether | Roche Vitamins Inc. Parsippany, NJ | 0.25 | 0.01 - 3 |
| Polyquaternium-7 | Ondea Nalco Center, Naperville, IL | 1 | 0.01 - 5 |
| Amodimethicone | Dow Corning Corporation, Midland, Michigan | 0.05 | 0.01 - 5 |
| Castor Isostearate Succinate | Zenitech LCC, Riverside, CT | 0.1 | 0.1- 3 |
| Dimethiconol (and) TEA Dodecylbenzenesulfo nate | Dow Corning Corporation, Midland, Michigan | 0.75 | 0.01 - 7 |
| Sodium Benzotriazolyl Butylphenol Sulfonate | Ciba, High Point, NC | 0.01 | 0.001 - 0.3 |
| Sweet Almond (Prunus Amygdalus Dulcis) Oil | Lipo Chemicals, Inc. Paterson, NJ | 0.1 | 0.01 - 3 |
| Phenoxyethanol | Dow Chemical USA Midland, MI | 0.5 | 0.01 - 2 |
| Fragrance | | 0.6 | 0.01 - 3 |

The above product was manufactured in the following manner. Guar hydroxypropyltrimonium chloride and Guar (Cyamopsis Tetragonoloba) gum were premixed and than slowly sprinkled into water. The gums were allowed to hydrate before citric acid and sodium trideceth sulfate and sodium lauroamphoacetate were added. While mixing the blend was heated up to 70-75°C. At this temperature methylparaben, propylparaben and cetyl alcohol were added and mixed until completely dissolved. The batch temperature was then reduced to 55-60°C and Cocamide MEA, Sodium Chloride, Meadowfoam (Limnanthes Alba) Seed Oil, Olive (Olea Europaea) Fruit Oil, Sweet Almond (Prunus Amygdalus Dulcis) Oil and Panthenyl Ethyl Ether were added. The mixture was mixed until it became uniform. After reducing the temperature to 50-55°C Polyquaternium-7, Amodimethicone and Castor Isostearate Succinate were added. After mixing the temperature was lowered to 40-45°C and the following ingredients were added one at the time: Dimethiconol (and) TEA Dodecylbenzenesulfonate, Sodium Benzotriazolyl Butylphenol Sulfonate, Phenoxyethanol and the fragrance. The blend was mixed until a uniform product was obtained.

### Example 2:

Table II an example of a formulation for a leave-in hair conditioning cream.

**Table II**

| **Component INCI Name** | **% W/W Range** |
|---|---|
| Water | 50-98 |
| Glycerin | 0.1 - 20 |
| Dimethicone | 0.1 - 10 |
| Polyquaternium 37 (and) Propylene Glycol Dicaprylate/Dicaprate (and) PPG-1 Trideceth-6 | 0.1 - 5 |
| Cetrimonium Chloride | 0.1 - 5 |
| Behenyl Alchol | 0.5 - 5 |
| Behentrimonium Chloride | 0.5 - 10 |
| Phenyl Trimethicone | 0.05 - 5 |
| Ethylhexyl Methoxycinnamate | 0.05 - 5 |
| Avobenzone | 0.01 - 3 |
| Ethylhexyl Isononanoate | 0.1 - 10 |
| Cyclopentasiloxane (and) Cyclohexasiloxane | 0.1 - 10 |
| Meadowfoam (Limnanthes Alba) Seed Oil | 0.1 - 10 |
| Methylparaben | 0.05 - 0.5 |
| Propylparaben | 0.01 - 0.5 |
| Olive (Olea Europaea) Fruit Oil | 0.01 - 10 |
| Sweet Almond (Prunus amygdalus dulcis) Oil | 0.001 - 10 |
| Fragrance | 0.05 - 1 |
| Sodium Hydroxide | 0.005 - 0.1 |

### Example 3:

Table III an example of a formulation for a rinse-off conditioner.

**Table III**

| **Component INCI Name** | **% W/W Range** |
|---|---|
| Water | 50 - 98 |
| Hydroxyethylcellulose | 0.01 - 1 |
| Dimethicone | 0.1 - 10 |
| Cetyl Alcohol | 0.5 - 10 |
| Behentrimonium Chloride | 0.1 - 10 |
| Stearamidopropyl Dimethylamine (and) Glycol Stearate (and) Ceteth-2 | 0.1 - 10 |
| Behentrimonium Methosulfate (and) Cetearyl Alcohol | 0.1 - 10 |
| Trimethylsilylamodimethicone | 0.05 - 5 |
| Behenyl Alcohol | 0.5 - 10 |
| Ethylhexyl Isononanoate | 0.5 - 15 |
| Meadowfoam (Limnanthes Alba) Seed Oil | 0.1 - 10 |
| Cyclopentasiloxane (and) Cyclohexasiloxane | 0.5 - 10 |
| Phenoxyethanol | 0.1 - 1 |
| Methylparaben | 0.05 - 0.5 |
| Propylparaben | 0.05 - 0.5 |
| Panthenol | 0.05 - 5 |
| Citric Acid | 0.01 - 0.5 |
| Olive (Olea Europaea) Fruit Oil | 0.01 - 5 |
| Sweet Almond (Prunus amygdalus dulcis) Oil | 0.001 - 5 |
| Fragrance | 0.05 - 1 |
| Mica (and) Titanium Dioxide (and) Iron Oxide | 0.01 - 0.5 |

### Example 4:

Table IV an example of a formulation for a rinse off conditioner.

**Table IV**

| **Component INCI Name** | **% W/W Range** |
|---|---|
| Purified Water | Qs to 100 |
| Cyclomethicone | 3 - 10 |
| Behenyl Alcohol | 1 - 10 |
| Behentrimonium Chloride | 0.5 -10 |
| Behentrimonium Methosulfate (and) Cetearyl Alcohol | 0.5 - 10 |
| Stearamidopropyl Dimethylamine (and) Glycol Stearate (and) Ceteth - 2 | 0.5 - 10 |
| Cetyl Alcohol | 0.5 - 10 |
| Dimethicone | 0.5 - 10 |
| Amodimethicone | 0.5 - 5 |
| Cetrimonium Chloride | 0.1 - 5 |
| Hydroxyethylcellulose | 0.1 - 1 |
| Limnanthes Alba(Meadowfoam) Seed Oil | 0.01 -10 |
| Fragrance | 0.05 - 5 |
| Methylparaben | 0.05 - 0.5 |
| Citric Acid | 0.01 - 1 |
| Propylparaben | 0.01 - 0.5 |
| Sweet Almond Oil | 0.001 - 10 |
| Panthenol (and) purified water | 0.01 - 10 |
| Mica(and)Titanium Dioxide(and) Iron Oxide | 0.01 - 0.5 |
| Olea Europaea(Olive) Fruit Oil | 0.01 -10 |

### Example 5:

Table V an example of a formulation for a leave-in hair conditioning foam product.

**Table V**

| **Component INCI Name** | **% W/W Range** |
|---|---|
| | |
| Water | qs to 100 |
| Polyquaternium-44 (and) water | 0.1 to 15 |
| Betaine | 0.1 to 5 |
| Panthenol (and) water | 0.1 to 5 |
| Panthenyl Ethyl Ether (and) Cocamidopropyl Hydroxysultaine (and) water | 0.1 to 2 |
| Hydrolyzed Vegetable Protein PG-Propyl Silanetriol (and) water | 0.1 to 5 |
| Cetrimonium Chloride (and) water | 0.1 to 1.5 |
| Behentrimonium Methosulfate and Cetearyl Alcohol | 0.1 to 5 |
| Avobenzone | 0.1 to 1 |
| Octinoxate | 0.01 to 1 |
| Meadowfoam (Limnanthes Alba) Seed Oil | 0.01 to 1 |
| Fragrance | 0.01 to 1 |
| Olive (Olea Europaea) Fruit Oil | 0.01 to 1 |
| Sweet Almond (Prunus Amygdalus Dulcis) Oil | 0.01 to 1 |
| Phenyl Trimethicone | 0.1 to 5 |
| Phenoxyethanol (and) Methylparaben (and) Ethylparaben (and) Butylparaben (and) Propylparaben | 0.3 to 1.5 |
| Polysorbate 20 | 0.1 to 5 |
| PPG-5-Ceteth-20 | 0.1 to 2 |

The above formulation can be added into an aerosol container having a 0.020" nozzle, with a propellant (such as isobutene).

### Example 6:

Table VI an example of a formulation for a hair shin treatment.

**Table VI**

| **Component INCI Name** | **% W/W Range** |
|---|---|
| Cyclopentasiloxane (and) Dimethiconol | 30 -90 |
| Cyclopentasiloxane (and) Cyclohexasiloxane | 15 - 50 |
| Hexamethyldisiloxane | 5 -25 |
| Pheny Trimethicone | 1 - 5 |
| Octyl Palmitate | 1 - 5 |
| Cyclopentasiloxane(and) Aminopropyldimethicone | 0.1 - 1 |
| Olive (Olea Europaea) Fruit Oil | 0.05 - 1 |
| Meadowfoam (Limnanthes Alba) Seed Oil | 0.05 - 1 |
| Fragrance | 0.01 - 1 |
| Sweet Almond Oil | 0.05 - 1 |
| Ethylhexyl Methoxycinnamate | 0.05 - 0.5 |
| BHT | 0.01 - 0.5 |
| Butyl Methoxydibenzoylmethane | 0.01 - 0.5 |

### Example 7:

Table VII an example of a formulation for a product for treatment of the hair prior to cleansing.

**Table VII**

| **Component INCI Name** | **% W/W Range** |
|---|---|
| Water | Qs to 100 |
| Hydroxyethylcellulose | 0.1 to 2 |
| Guar Hydroxypropyltrimonium Chloride | 0.05 to 1 |
| Cetrimonium Chloride (and) Water | 0.1 to 5 |
| Polyquaternium-7 (and) water | 0.1 to 10 |
| Hydrolyzed Vegetable Protein PG-Propyl Silanetriol (and) water | 0.1 to 10 |
| Sodium Benzotriazolyl Butylphenol Sulfonate | 0.01 to 0.2 |
| Disodium EDTA | 0.1 to 0.5 |
| PPG/PEG-18 Dimethicone | 0.1 to 5 |
| Glycerin | 0.1 to 30 |
| Betaine | 0.1 to 5 |
| Dyes | 0.01 to 1 |
| Panthenol (and) Water | 0.1 to 5 |
| Propylene Glycol (and) Water (and) Aloe Barbadensis Leaf Extract | 0.1 to 5 |
| Propylene Glycol (and) Water (and) Matricaria (Chamomilla Recutita) Flower Extract | 0.1 to 5 |
| PPG-5-Ceteth-20 (and) Polysorbate 20 | 0.1 to 3 |
| Fragrance | 0.1 to 2 |
| Olive (Olea Europaea) Fruit Oil | 0.001 to 5 |
| Sweet Almond (Prunus Amygdalus Dulcis) Oil | 0.001 to 5 |
| Meadowfoam (Limnanthes Alba) Seed Oil | 0.001 to 5 |
| BHT | 0.01 to 0.1 |
| Phenoxyethanol (and) Methylparaben (and) Ethylparaben (and) Propylparaben (and) Butylparaben | 0.3 to 1.5 |

### Example 8:

Various hair conditioning agents were tested for their ability to penetrate into human hair. Secondary ion mass spectrometry (SIMS) in combination with a time of flight (TOF) mass spectrometer was used to determine hair penetration. Time-Of-Flight Secondary Ion Mass Spectrometry (TOF-SIMS) makes use of the secondary ion mass spectra (of atomic species or low molecular weight fragments), which are formed when the sample surface (e.g., surface of hair fiber cross sections) is bombarded with a positively charged gallium ion beam. The positive and negative ion mass spectra of the sample were obtained by the time-of-flight method. See, e.g., Ruetsch, et al., J. Cosmet. Sci., 52, 169-184 (2001). The Gallium gun, which emits a pulsed primary ion beam (accelerating voltage of 25 kV), was used in the study.
- Instrumentation: PHI TFS-2000 (Physical Electronics USA, Chanhassen, MN 55317)
- Primary ion beam: ⁶⁹GA⁺ liquid metal ion beam (bunched)
- Primary beam voltage: 18 kV⁺ ions, 12 kV⁻ ions
- Primary ion current (DC): 600 pA
- Nominal analysis region: (120 µm)², (180 µm)²
- Charge neutralization: yes
- Post acceleration: 8000 V
- Masses blanked: None
- Energy filter: No
- Contrast diaphragm: No

The protocol was as follows. Dark brown European hair obtained from DeMeo Brothers, New York, NY was bleached for 30 minutes with alkaline 6% hydrogen peroxide (adjusted to pH 10.2 with ammonium hydroxide). The following hair conditioning agents used for the study: olive oil (Cropure® Olive #OL2-193E, Croda Inc., Parsipanny, NJ), almond oil (Cropure® Almond #AO2-196, Lipo Chemicals, Paterson, NJ); avocado oil (Cropure® Avocado #AV2-187, Croda Inc.); Meadowfoam Seed Oil (Botagenics, Inc., Northridge, CA); PEG-7 Olivate (B&T SRL Biologia & Tecnologia, Milan, Italy); Sweet Almond Milk (containing Almond Proteins) (Mandor Lat, Sinerga, Rue dela Procession, France), Ceramide A2 (containing PEG-8/SMDI Copolymer and Palmitoyl Myristyl Serinate; from Sederma, Parsipanny, NJ); Sunflower Oil (Desert Whale Jojoba Co, Tuczon, AZ), and Jojoba Oil (Desert Whale Jojoba Co.).

Nine small bundles of bleached hair fibers were treated by gently massaging the hair with one of the above nine hair conditioning agents and allowing the hair to rest on a hair slide for 24 hours at approximately 32°C. The treatments were followed by thorough 1 minute rinsing in very warm running water, while stroking the small hair bundle with a gloved hand in the with-scale direction, followed by air-drying at room temperature. This was done to remove extraneous oils from the fiber surface. Bleached hair fibers (serving as controls) were rinsed well, blotted, and dried at room temperature.

Sample preparations for TOF-SIMS were prepared as follows. Small amounts of the conditioning agents were deposited on clean silicon wafers at ambient temperature. Bleached and bleached/product-treated hair fibers were cross-sectioned each with a clean stainless steel blade and mounted in small holders with the cross-sections facing the spectrometer. Positive and negative TOF SIMS mass spectra were acquired from several locations on the nine wafers coated with the standards as well as from the cross-sectioned surface of the bleached hair, to identify the characteristic peaks of the standards. Unique, characteristic positive ions of the nine compounds (not inherent in the bleached hair cross section) were established and selected. Positive spectra and images of the characteristic positive ions were then collected from cross sections of the treated hair fibers. Data were collected within the static limit, therefore, low molecular fragments are indicative of species existing on the surfaces prior to analysis.

Positive and negative spectra of bleached hair and the nine standards were obtained and compared. The positive spectra of the standards showed unique, characteristic peaks suited for imaging. Therefore, the positive spectra were chosen for this study. Positive spectra and images of the characteristic positive ions were collected on the surface of the product-treated fiber cross sections. The results indicate that Avocado Oil, Olive Oil, and PEG-7 Olivate penetrate all the way to the center of the fiber or inner cortex. Meadow foam seed oil was found to have penetrated past the surface to the outer cortex, but not through to the center of the hair fiber cross section. Almond oil penetrated slightly into the outer surface layer, and the PEG-8/SMDI Copolymer, Palmitoyl Myristyl Serinate, Almond Proteins, Almond oil, Sunflower Oil and Jojoba Oil were not detected in the cross section of treated hair. Rather, they remain on the surface.

It is understood that while the invention has been described in conjunction with the detailed description thereof, that the foregoing description is intended to illustrate, and not limit the scope of the invention, which is defined by the scope of the appended claims. Other aspects, advantages, and modifications are within the claims.

## Claims

1. A composition for the application to the hair, said composition comprising:
(i) a first hair conditioning agent, wherein said first hair conditioning agent is selected from the group consisting of avocado oil, apricot kernel oil, olive oil, sesame oil, coconut oil and PEG-7 olivate;
(ii) a second hair conditioning agent, wherein said second hair conditioning agent is meadowfoam seed oil; and
(iii) a third hair conditioning agent, wherein said third hair conditioning agent is selected from the group consisting of PEG-8/SMDI copolymer, palmitoyl myristyl serinate, jojoba oil, almond oil, almond proteins, and sunflower oil.

2. A composition of claim 1, wherein said first hair conditioning agent is olive oil.

3. A composition of claim 1, wherein said first hair conditioning agent is avocado oil.

4. A composition of claim 1, wherein said third hair conditioning agent is almond oil.

5. A composition of claim 2 or 3, wherein said third hair conditioning agent is almond oil.

6. A method for conditioning the hair, said method comprising applying to said hair a composition of claim 1.

7. A method of claim 6, wherein said first hair conditioning agent is olive oil.

8. A method of claim 6, wherein said first hair conditioning agent is avocado oil.

9. A method of claim 6, wherein said third hair conditioning agent is almond oil.

10. A method of claim 7 or 8, wherein said third hair conditioning agent is almond oil.
